# EUROPÄISCHE PATENTSCHRIFT

(11) **EP 1 269 933 B1**
(45) Veröffentlichungstag und Bekanntmachung des Hinweises auf die Patenterteilung: **17.02.2010**
(21) Anmeldenummer: 02013825.1
(22) Anmeldetag: 21.06.2002
(51) Int. Cl.: A61C 8/00, A61C 3/02, A61B 17/16

(54) **Bohrkopf**
Drill head
Tête de perçage

(30) Priorität: 21.06.2001 DE 10129948
(43) Veröffentlichungstag der Anmeldung: 02.01.2003
(73) Patentinhaber: Edlhuber, Christian, 82481 Mittenwald (DE)
(72) Erfinder: Edlhuber, Christian, 82481 Mittenwald (DE)
(74) Vertreter: Winter, Brandl, Fürniss, Hübner Röss, Kaiser, Polte Partnerschaft Patent- und Rechtsanwaltskanzlei

(56) Entgegenhaltungen:
- EP-A- 0 843 987
- DE-A- 19 944 796
- US-A- 2 525 669

## Beschreibung

Die vorliegende Erfindung betrifft einen Bohrkopf mit einem Bohrer, insbesondere einem Implantatbettvorbohrer.

In der nachfolgenden Beschreibung sei unter dem Begriff "proximal" "patientenseitig" verstanden. Der Begriff "distal" soll "operateurseitig" bedeuten.

Das Gebiet der vorliegenden Erfindung liegt bei der Zahnprothetik, insbesondere dem Einsetzen von Zahnimplantaten in vorhandene Zahnlücken. Häufig betrifft das Einsetzen von Zahnimplantaten im Oberkiefer Positionen mit nur geringem Knochenangebot, um das Implantat sicher und dauerhaft zu verankern. Dies ist insbesondere der Fall bei Positionen, in welchen die Wurzeln der ursprünglichen eigenen Zähne von der Kieferhöhle überdeckt werden. Als Operationsmethode hat sich hierbei etabliert, das Lumen der Kieferhöhle zu verkleinern und das Implantatbett in die Kieferhöhle zu erstrecken.

Im Stand der Technik wird beispielsweise die im folgenden kursorisch beschriebene Osteotom-Sinusbodenelevation als chirurgisches Procedere angewendet:

Die Voraussetzung für eine Osteotom-Sinusbodenelevation ist in der Regel eine minimale Distanz von 5-6 mm zwischen Kieferkamm und Sinusboden im Bereich der zukünftigen Implantatposition. Nach lokaler Anästhesie wird ein vestibulär gestielter Mukoperiost-Lappen (Zahnfleischdistanzschnitt) mobilisiert und die Knochenstruktur wird freigelegt.

Zum Erzielen einer prothetisch optimierten Implantatachse wird häufig eine Bohrschablone, z.B. OP-Schablone / CT-Schiene eingesetzt. Dies erleichtert die Markierung der prospektiven Implantatlokalisation mit einem 2 mm Rosenbohrer. Mit einem spiralförmigen Pilotbohrer von 2 mm Durchmesser wird anschließend die primäre Aufbereitung der Knochenkavität bis ca. 2 mm vor den Sinusboden begonnen. Die weitere Formung des Implantatbettes erfolgt mit am proximalen Ende stempelförmigen Osteotomen von aufsteigenden Durchmessern. Das Osteotom wird mit seinem konusförmig zulaufenden, stempelförmigen Arbeitsende (proximales Ende) mit z.B. 2,2 mm Durchmeser in prothetisch korrekter Achsrichtung eingeführt und mit schwachen Hammerschlägen bis zum apikalen Stopp vorangetrieben. Das Instrument wird daraufhin mit drehenden Bewegungen zurückgezogen.

Die weitere Aufbereitung erfolgt konsekutiv mit Osteotomen ansteigender Durchmesser bis zur entsprechenden Knochentiefe. Die perfekte Abstimmung der Instrumentendurchmesser ermöglicht dem Operateur einen geführten Aufbereitungsvorgang. Häufig sind Längenmarkierungen auf dem Osteotom angebracht, welche ein kontrolliertes Erweitern des Implantatbettes nach cranial sichern. Erst mit dem finalen Osteotom, z.B. mit einem Instrumentendurchmesser 2,7/3,2mm bei einem Implantatdurchmesser von 3,75 mm wird die verdichtete Knochenstruktur mit dem Sinusboden in Form einer Grünholzfraktur nach cranial eleviert. Dabei muss die Sinusschleimhaut (Schneider'sche Membran) intakt bleiben.

Nachfolgend wird in den Knochenschacht mehrmals Augmentationsmaterial mit dem entsprechenden Osteotom vorsichtig nach cranial weiter vorangetrieben und kondensiert. Die Endposition bemerkt der Operateur, wenn ein leichter elastischer Widerstand zu verspüren ist.

In das aufbereitete Knochenbett wird anschließend beispielsweise ein schraubenförmiges Implantat eingedreht bzw. ein zylindrisches oder konisches Implantat eingeklopft. Nach Abdeckung des Implantates mit einer Deckschraube wird ein dichter Weichteilverschluss vorgenommen.

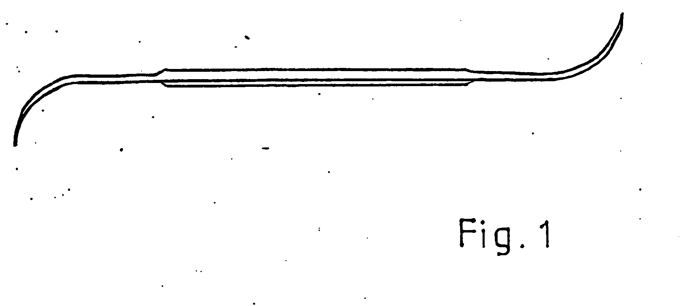

Zum Ablösen der Sinusschleimhaut vom knöchernen Boden der Kieferhöhle ist ferner eine Sinusliftkürette der Firma STOMA [STOMA Dentalsysteme GmbH & Co KG, Emminger Strasse 39, D-78576 Emmingen-Uptingen] bekannt, welche in Fig. 1 gezeigt ist. Darüberhinaus werden löffelförmige Instrumente verwendet, um die Sinusschleimhaut unter äußerster Vorsicht abzulösen und zu verdrängen.

Gerne würden Zahnärzte bei Bedarf Zahnimplantate häufiger einsetzen. Viele schrecken jedoch vor den möglichen Komplikationen zurück, die in der Hauptsache darin liegen, dass der Implantatbettbohrer den (bohrtechnisch) ungefährlichen Knochenbereich verläßt und anatomische Weichgewebestrukturen verletzt. Diese Verletzungen können insbesondere Auskleidungen von Körperhöhlen, wie beispielsweise die Schneider'sche Membran des Sinus maxillaris (Kieferhöhle), Nerven und Gefäße betreffen.

Ein besonderes Problem und hohe Anforderungen an die Geschicklichkeit des Operateurs stellt die Herstellung des Knochenkanals bis zum Kieferhöhlenboden dar, ohne dass die hauchdünne Auskleidung der Kieferhöhle bereits beim Bohren oder auch mittels des Osteotoms penetriert wird, da in der Regel nicht unter Sicht gearbeitet werden kann. Tritt trotz aller Vorsicht und Erfahrung des Operateurs dennoch eine Perforation der Schneider'schen Membran auf, geraten einerseits Bestandteile der Sinusmischflora in den Knochenbereich, was zu späteren Knochenentzündungen führen kann. Andererseits besteht die Gefahr, dass das Basismaterial, in der Regel aus einem Gemisch aus künstlichem Knochen, Eigenknochen und gegebenenfalls Eigenplasma, welches das spätere Implantatbett bilden soll, das Implantat nicht oder nicht ausreichend am Kieferhöhlenknochen hält.

Wegen dieses Komplikationsrisikos wurden im Stand der Technik komplexe und teure Navigationssysteme entwickelt, welche dem Operateur über den Umweg von Computer-Tomogrammen mit Bezugspunktschlüsseln, welche Laser-gestützt und/oder Holographie-gestützt simultan Angaben über die Position des Bohrers vermitteln. Diese Systeme sind jedoch einerseits sehr teuer (zwischen 100.000 und 1.000.000 Euro) und sind anderseits nicht geeignet ein weiteres Problem zu beseitigen:

Häufig findet der Zahnarzt in den Bereichen, in denen aus prothetischen Gründen eine Implantat-Insertion wünschenswert wäre, ein nicht ausreichendes oder nur durch komplikationsreiches Vorgehen nutzbares Knochenangebot. So kann häufig im hinteren Oberkiefer nur ein Implantat gesetzt werden, wenn die Kieferhöhle verkleinert wird. Dabei wird die hauchdünne innere Auskleidung der Kieferhöhle, die Schneider'sche Membran, mit entsprechenden löffelförmigen Instrumenten unter äußerster Vorsicht vom Knochen abgelöst und verdrängt. Der zusätzlich gewonnene Raum wird mit einem Gemisch aus künstlichem Knochen, Eigenknochen und evtl. Eigenplasma, das um den in die Kieferhöhle ragenden Implantatteil geschlichtet wird, gefüllt. Hier unterscheidet man zwischen dem
a) externen Sinuslift und dem
b) internen Sinuslift.

Beim externen Sinuslift wird von der Umschlagfalte in die Kieferhöhlen-Seitenwand ein Fenster ausgeschnitten, das zur Seite geschwenkt wird und somit Zugang gewährt, um die oben beschriebenen Manipulationen unter Sicht durchführen zu können. Der Umfang dieses Eingriffes bedeutet grosse postoperative Belastung für den Patienten.

Bei dem bereits eingangs erläuterten internen Sinuslift tastet sich der Operateur mit einem Implantatbett-Bohrer an den Boden der Kieferhöhle heran. Die oben erwähnten Navigations-Systeme können zwar bis zu diesem Punkt unterstützen, stellen jedoch für das weitere Vorgehen keine weitere Hilfe dar.

So kann der Bohrer selbst bei genauer Kenntnis der Lage beim Durchbruch durch die Corticalis in die Kieferhöhle "einbrechen" und hierbei die Kieferhöhlenschleimhaut penetrieren, da an diesem kritischen Punkt mehrere nachteilige Faktoren aufeinandertreffen:
1.) Die Corticalis besitzt eine größere Knochendichte, welche größeren Druck zur Überwindung erfordert.
2.) Dieser erhöhte Widerstand bricht plötzlich weg, wenn der Bohrer diese Schicht durchdringt.
3.) Die Anforderungen an die Reaktionszeit des Operateurs sind sehr hoch, um den plötzlich verringerten Gewebswiderstand zu spüren und den Bohrer rechtzeitig zurückzuziehen.
4.) Selbst bei ausgezeichneter Reaktionszeit des Operateurs kann es dieser häufig nicht schaffen, auch die Drehbewegung des Bohrers so schnell zu beenden, dass sich dieser nicht aufgrund des Drills von alleine nach vorne ziehen würde.

Unter dem eingangs beispielhaft erwähnten Operationsverfahren drängt der Operateur mittels Einsatz von Osteotomen nach gelungener Präparation eines Knochenkanals durch die mundhöhlenseitige Corticalis, Sporngiosa und kieferhöhlenseitige Corticalis die Schneider'sche Membran vom Knochen ab, ohne diese einzureißen. Dies erfolgt ohne direkte Sicht und erfordert deshalb grosses Fingerspitzengefühl bei Anwendung der doch relativ grossen Kräfte, die nötig sind, um Restknochen und Kieferhöhlenauskleidung lösen und weit genug bewegen zu können. Dabei können scharfkantige Knochenstücke oder Osteotome selbst größte Sorgfaltsbemühungen des Operateurs zunichte machen.

Navigations-Systeme, z B. "RoboDoc", "NewTom DVT 900011", "med 3 D", "Hip Nav", "Sim/Plant" usw. können - wie oben schon beschrieben - dem Operateur zwar simultan einen Bericht über die Lage des Bohrers geben, sind jedoch neben den hohen Anschaffungskosten nicht geeignet, die Probleme zu beherrschen, welche auftreten, wenn der Operateur durch Bohren zwar eine Verbindung vom dichteren zum weniger dichten Medium schaffen will, jedoch nicht zu weit ins weniger dichte Medium vordringen will.

Die EP 0 843 987 A1 bzw. die US-A-2,525,669 beschreiben chirurgische Bohrer bzw. Trepane, bei denen Fühlmittel am Bohrkopf vorhanden sind, welche bei Über- oder Unterschreiten einer bestimmten axialen Schwellenwert- oder Grenzkraft den sich drehenden Bohrer von den Antriebsmitteln entkoppeln. Diese Vorrichtungen sind hochkompliziert, teuer, schwer zu sterilisieren und aufgrund des komplexen Aufbaus störanfälliger als herkömmliche Bohrköpfe.

Die DE 199 44 796 A - von der vorliegende Erfindung ausgeht - beschreibt eine Fräse zur zahnärztlichen Implantation unter Schutz der Kieferhöhle, bei der koaxial im Inneren des Bohrers oder Fräskopfes ein Taststift oder eine Fühlerspitze angeordnet ist. Die Fühlerspitze steht unter Federspannung derart, dass sie aus der Bohrerspitze herausgedrängt wird, wenn sie auf keinen Widerstand oder einen Widerstand trifft, der von der Federkraft überwunden werden kann. Hierbei soll "manuell ertastbar" sein, wenn die federbelastete Fühlerspitze unter der Kraft der Feder aus der Bohrerspitze heraustritt, etwa dann, wenn die kieferhöhlenseitige Corticalis durchbrochen wird. Ein Austauschen der Feder, welche die Fühlerspitze belastet, soll eine Anpassung an unterschiedliche Gegebenheiten ermöglichen.

Als Hauptnachteil wird gesehen, dass diese mit der Fühlerspitze versehene Fräse nach wie vor ein erhebliches Geschick und eine äußerst geschulte Haptik des Operateurs bedingt: das Auf- oder Ausbohren eines Kieferknochens zur Implantatvorbereitung erfordert einen nicht unerheblichen Kraftaufwand und bislang war der Operateur bei dieser Behandlung äußerst gefordert, um ein Durchstossen bis in die Kieferhöhle zu vermeiden.

Der Gegenstand der DE 199 44 796 A vermag dieses Problem - wenn überhaupt - dann nur sehr bedingt zu mindern, da das federbelastete Treiben der Nadel durch eine dünne Knochenlamelle oder durch die Kieferschleimhaut nur dann im Sinne der DE 199 44 796 A "manuell ertastbar" sein kann, wenn der Operateur hierauf entsprechend sensibilisiert ist. Gleichwohl kann es durchaus vorkommen, dass der sich zwangsläufig nur durch eine geringfügige Änderung im Bohrverhalten äußernde, jedoch entscheidende Vorgang, nämlich dass die Fühlerspitze die Kieferschleimhaut penetriert, in dem allgemeinen Bohrvorgang "untergeht", da nur eine geringfügige Änderung der vorherrschenden hohen Handkraft bei dieser Penetration auftritt.

Ausgehend vom Stand der Technik gemäß der DE 199 44 796 A ist es daher Aufgabe der vorliegenden Erfindung, ein Instrumentarium zum leichteren und sicheren Durchführen von Implantationen in anatomisch kritischen Bereichen, wie beispielsweise im Bereich des Oberkiefers in der Nähe der Kieferhöhlen zur Verfügung zu stellen.

Die Lösung dieser Aufgabe erfolgt durch die im Anspruch 1 angegebenen Merkmale.

Allgemein gesagt, es wird ein Implantatbettvorbohrer zum Knochenfräsen zur Vorbereitung des Einsetzens von Zahnimplantaten vorgesehen, welcher als Einsatz in einem mit Antrlebs- und Kühlmitteln ausgestatteten Bohrkopf vorgesehen ist. Der Bohrer hat eine äußere Bohrerspitze, in deren Innerem eine koaxial gelagerte Fühlerspitze angeordnet ist. Die Fühlerspitze ist in axialer Richtung gegenüber der Spitze des Bohrers verschiebbar und mit einem vorbestimmten einstellbaren Schwellenwert kraftbeaufschlagt. Beim Überschreiten des Schwellenwertes wird die Fühlerspitze in die äußere Bohrerspitze hineingedrängt und bei Unterschreiten dieser Schwellenwertkraft tritt die Fühlerspitze aus der äußeren Bohrerspitze heraus. Hierdurch wird es einem Operateur mit einfachen baulichen Mitteln möglich gemacht, die jeweiligen beim Bohrvorgang herrschenden Kraftverhältnisse visuell festzustellen. Durch die gegenüber der Bohrerspitze axial verschiebbaren Fühlerspitze kann unter Zuhilfenahme einfachster Mittel dem Operateur der Übergang von hartem zu weichem Knochen- oder Gewebematerial und umgekehrt angezeigt werden. Weiterhin kann die bewegliche Fühlerspitze Kontakte betätigen, um die Bohrerspitze bei Bedarf abzustoppen, es können Dehnungsmessstreifen verwendet werden, welche bei einem entsprechenden Abgriff über einen Monitor Aussage über den momentanen Bohrzustand erlauben etc. Weiterhin kann über die Fühlerspitze auch eine Kühl- oder Spülflüssigkeit, insbesondere physiologische Kochsalzlösung zugeführt werden.

Da für den Operateur der Übergang von hartem zu weichem Knochen- oder Gewebematerial und umgekehrt klar erkennbar ist, kann er die Handhabung des Bohrers hierauf entsprechend abstimmen, insbesondere den Bohrvorgang abbrechen, wenn das der Bohrerseite abgewandte Ende der Fühlerspitze anzeigt, dass die Fühlerspitze Gewebematerial, beispielsweise die Corticalis, erreicht oder durchdrungen hat.

Bei dem erfindungsgemäßen Bohrer kann eine Schwellenwertkraft der Fühlerspitze durch eine Druckbeaufschlagung mittels eines Fluids erfolgen, welches beispielsweise eine Flüssigkeit, insbesondere physiologische Kochsalzlösung sein kann.

Die Druckbeaufschlagung kann auch bevorzugt mittels einer Kolbenpumpe erfolgen.

Weiterhin kann die Schwellenwertkraft der Fühlerspitze auch durch eine inhärente elastische Rückstellkraft erzeugt wird.

Bevorzugt tritt das der Bohrerseite abgewandte Ende der Fühlerspitze beim Hineindrängen der Fühlerspitze in die äußere Bohrerspitze aus dem Bohrkopf heraust und tritt umgekehrt beim Herauschieben der Fühlerspitze aus der äußeren Bohrerspitze in den Bohrkopf ein.

Der Bohrer im Bohrkopf gemäß der vorliegenden Erfindung eignet sich hervorragend zum kontrollierten und sicheren Knochenfräsen zur Vorbereitung des Einsetzens von Zahnimplantaten, welche in der Kieferhöhle, jedoch ohne Zerstörung der Schneider'schen Membran, verankert werden müssen. Das erfindungsgemäße Vorbohrerprinzip kann natürlich auch bei der Durchführung anderer chirurgischer Handlungen Verwendung finden, bei denen es von Vorteil ist, unterschiedliche Dichten und somit Widerstandskräfte von Geweben zu erspüren. Insbesondere der Schutz von gefährdeten Strukturen im Knochen wie Nerven und Gefäße sei als weiteres Indikationsgebiet erwähnt: So besteht bei der Implantation im distalen Bereich des Unterkiefers die Gefahr, den nervus mandibularis zu verletzen, wenn der Bohrer zu tief vordringt, mit der Folge von Gefühlsverlust und Funktionseinbuße im Bereich der Unterlippe und am Kinn. Der Vorbohrer kann hier das Gefäß-/Nervenbündel genauso aufspüren helfen wie im Oberkiefer die Schneider'sche Membran. Darüber hinaus gibt es im Spezialfall der "zahnärztlichen Implantologie" nicht nur die Erfordernis, im Bereich der Kieferhöhle zu augmentieren. Genauso kann es im Bereich von Einziehungen und Unterschnitten von Alveolarkamm und Kieferform vorteilhaft sein, das Periost mittels des erfindungsgemäßen Vorbohrers aufzuspüren und dort im Sinne einer Elevation Knochen aufzubauen. Die Unversehrtheit des Periostes ist hier Garant für den Erfolg. Bei mangelnder Kieferkörperhöhe im Unterkiefer ist nach eben benannten Vorgehen auch eine submentale Augmentation vorteilhaft.

Abgesehen von medizinischen Einsatzmöglichkeiten kann der erfindungsgemäße Bohrkopf sogar auch im technischen Bereich Anwendung finden, wenn sensible Strukturen aufgespürt werden müssen, ohne diese zu zerstören, z.B. beim Bohren in mehrschichtigen Strukturen, wie etwa umhüllten Leitungen, Rohrleitungen und elektrischen Leitungen in Hauswänden, insbesondere Ziegel- und Trockenbauwänden.

Weitere Vorteile und Merkmale der vorliegenden Erfindung ergeben sich aufgrund der Beschreibung anhand der Zeichnung.

Es zeigt:
Fig. 1 eine Sinusliftkürette des Standes der Technik;
Fig. 2 eine Ansicht eines Zahnimplantates (links) und eines handelsüblichen Implantatbett-Bohrers (rechts) der Firma FRIATEC, Modell "Frialit-2 Synchro" eingespannt In einen Winkelstück-Triebkopf mit Kühlmediumzuführung von oben in das Zentrum der Fräse;
Fig. 3 eine Ansicht eines Implantatbettvorbohrers (rechts), der in einen handelsüblichen Winkelstück-Triebkopf eingesetzt ist mit eingefahrener Fühlerspitze und von oben zugeführtem Druckaufbau- und Druckmesssystem sowie einen handelsüblichen Implantatbett-Bohrer (mitte) und ein Zahnimplantat (links) - dieser Implantatbettvorbohrer und seine nachfolgende Beschreibung gehören nicht zum Umfang der vorliegenden Erfindung;
Fig. 4 eine Ansicht eines Implantatbettvorbohrers mit eingefahrener Fühlerspitze - dieser Implantatbettvorbohrer und seine nachfolgende Beschreibung gehören nicht zum Umfang der vorliegenden Erfindung;
Fig. 5 eine Ansicht eines Implantatbettvorbohrers (rechts), der in einen handelsüblichen Winkelstück-Triebkopf eingesetzt ist mit ausgefahrener Fühlerspitze und von oben zugeführtem Druckaufbau- und Druckmesssystem sowie einen handelsüblichen Implantatbett-Bohrer (mitte) und ein Zahnimplantat (links) - dieser Implantatbettvorbohrer und seine nachfolgende Beschreibung gehören nicht zum Umfang der vorliegenden Erfindung;
Fig.6 eine Ansicht eines Implantatbettvorbohrers mit ausgefahrener Fühlerspitze - dieser Implantatbettvorbohrer und seine nachfolgende Beschreibung gehören nicht zum Umfang der vorliegenden Erfindung;
Fig. 7 einen Längsschnitt durch einen Implantatbettvorbohrer mit eingefahrener Fühlerspitze - dieser Implantatbettvorbohrer und seine nachfolgende Beschreibung gehören nicht zum Umfang der vorliegenden Erfindung;
Fig. 8 einen Längsschnitt durch einen Implantatbettvorbohrer mit ausgefahrener Fühlerspitze - dieser Implantatbettvorbohrer und seine nachfolgende Beschreibung gehören nicht zum Umfang der vorliegenden Erfindung;
Fig. 9 eine schematische Gesamtansicht eines Bohrsystems, teilweise als Schnittansicht, eines Bohrers im Bohrzustand, kurz bevor er die kieferhöhlenseitige Corticalis erreicht, mit externer Beschaltung in schematischer Darstellung - dieses Bohrsystem und seine nachfolgende Beschreibung gehören nicht zum Umfang der vorliegenden Erfindung;
Fig. 10 eine schematische Gesamtansicht des Bohrsystems, teilweise als Schnittansicht, eines Bohrers im AUS-Zustand, durch automatisches Abschalten des Systems, wenn die Fühlerspitze plötzlich die kieferhöhlenseitige Corticalis durchdringt, mit externer Beschaltung in schematischer Darstellung - dieses Bohrsystem und seine nachfolgende Beschreibung gehören nicht zum Umfang der vorliegenden Erfindung;
Fig. 11 eine Ausführungsform der vorliegenden Erfindung in Gesamtansicht;
Fig. 12 die Ausführungsform von Fig. 11 in einem anderen Betriebszustand;
Fig. 13 eine Ansicht zur Veranschaulichung der Arbeitsweise der Ausführungsform von Fig. 11; und
Fig. 14 eine Ansicht entsprechend Fig. 13 in einem anderen Arbeitszustand.

Fig 1 zeigt eine Sinuskürette des Stand der Technik zum Abdrängen der Schneider'schen Membran mit auf beiden Seiten löffelförmigem Ende nach Art eines Spatels.

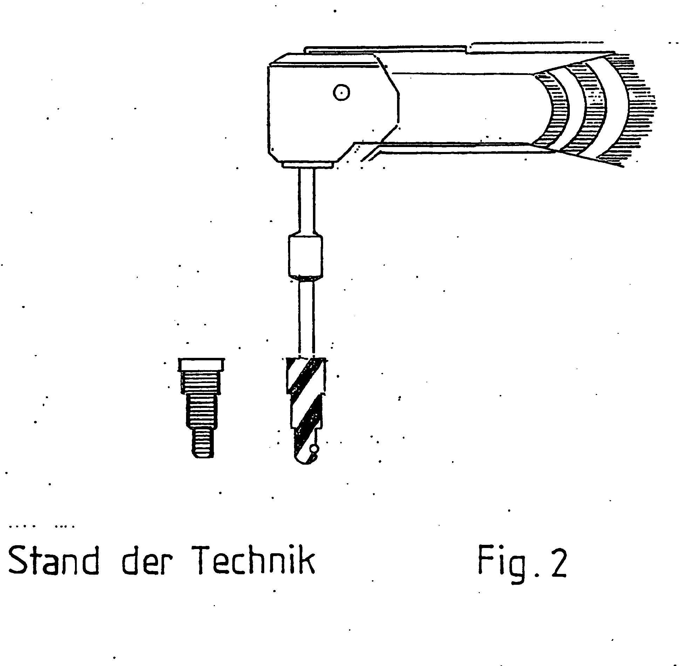

Fig. 2 zeigt ein modernes Implantat-System "Frialit-2 Synchro" der Fa. Friatic; links: Implantat; rechts Winkelstück-Triebkopf mit eingespanntem Implantatbett-Bohrer mit Kühlmediumzuführung von oben ins Zentrum der Fräse, wie es derzeit zur Implantationen von Zahnimplantaten eingesetzt wird. Implantate sind Metallstifte, die direkt In den Kieferknochen einzementiert werden. Darauf wird dann die sogenannte Suprakonstruktion gesetzt, ein künstlicher Zahn, ähnlich einer normalen Krone.

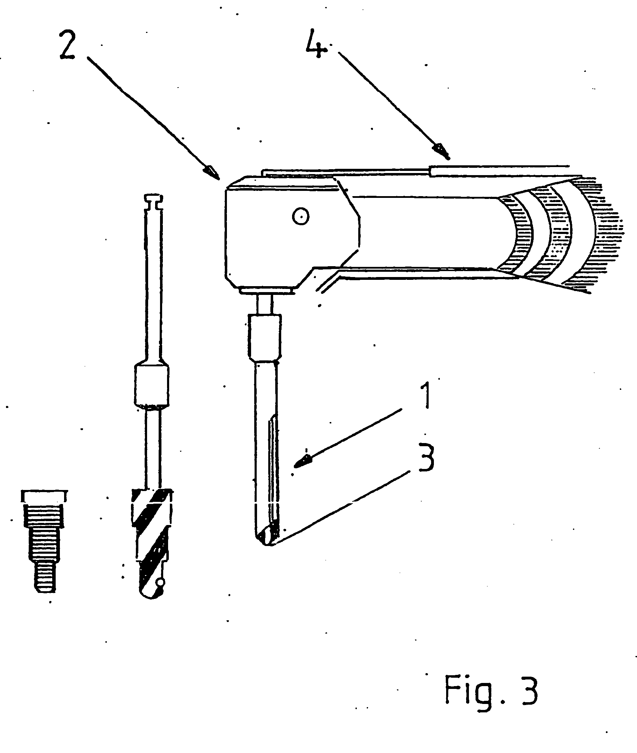

Die nachfolgenden Ausführungen unter Bezug auf die Figuren 3 bis 10 sind nicht Gegenstand der vorliegenden Erfindung. Sie betreffen vom Erfinder durchgeführte Modifikationen an Bohrköpfen nach dem Stand der Technik in dem Bestreben, diese technisch aufzurüsten und sich damit dem Ziel der vorliegenden Erfindung zu nähern. Vom Grundprinzip her entsprechen die Ergebnisse denjenigen der eingangs genannten EP 0 843 987 A1 bzw. US-A-2,525,669.

Gezeigt ist ein Bohrer 1 - im Beispielsfalle ein Implantabettvorbohrer - der in einen handelsüblichen Winkelstück-Triebkopf 2 eingesetzt ist mit eingefahrener Fühlerspitze 3 und von oben zugeführtem Druckaufbaü- und -messsystem 4. Die Fühlerspitze 3 befindet sich immer dann im eingefahrenen Zustand, wenn die Widerstandskraft des bearbeiteten Knochengewebes größer oder gleich der mittels einer nicht gezeigten elektronischen Steuerung voreingestellten Schwellenwertkraft ist. Diese Schwellenwertkraft wird an den Implantatbettvorbohrer durch Anlegen eines Fluiddruckes im Inneren des Implantatbettvorbohrers erzeugt. Im Beispielsfalle wird als Fluid physiologische NaCl-Lösung verwendet. Die Kochsalzlösung wird mit Hilfe einer computergesteuerten Präzisisionspumpe unter Druck gesetzt. Die Schwellenwert und IST-Wertdaten des an der Fühlerspitze 3 anliegenden Druckes werden von einem Rechner ausgewertet und in Echtzeit auf einem Monitor graphisch dargestellt.

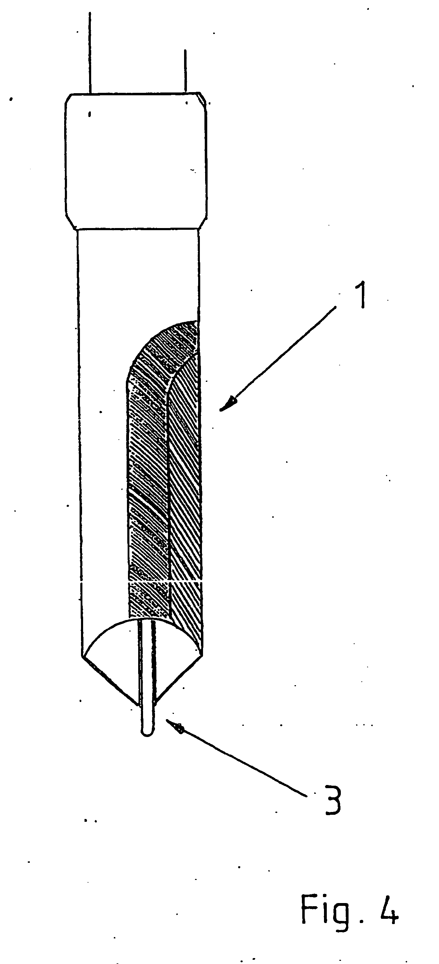

Fig. 4 zeigt den Bohrer der Fig. 3 in vergrößerter Darstellung.

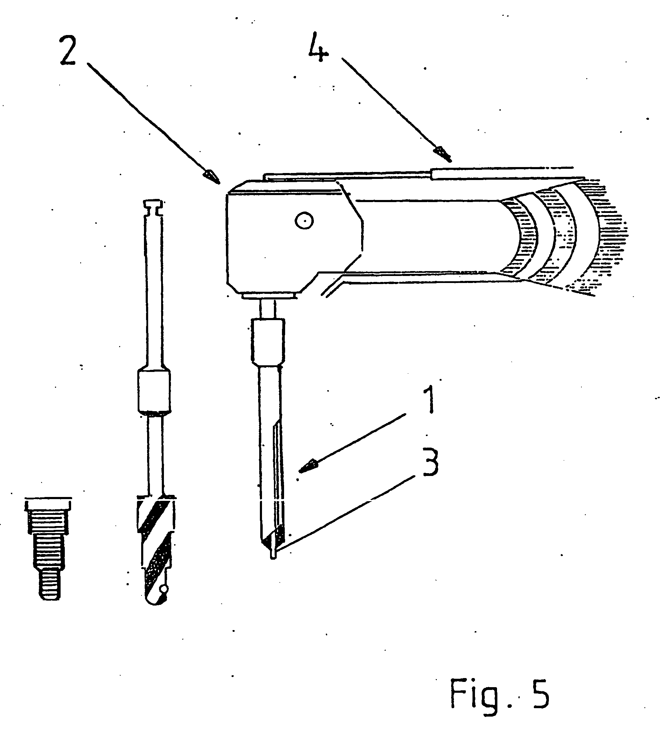

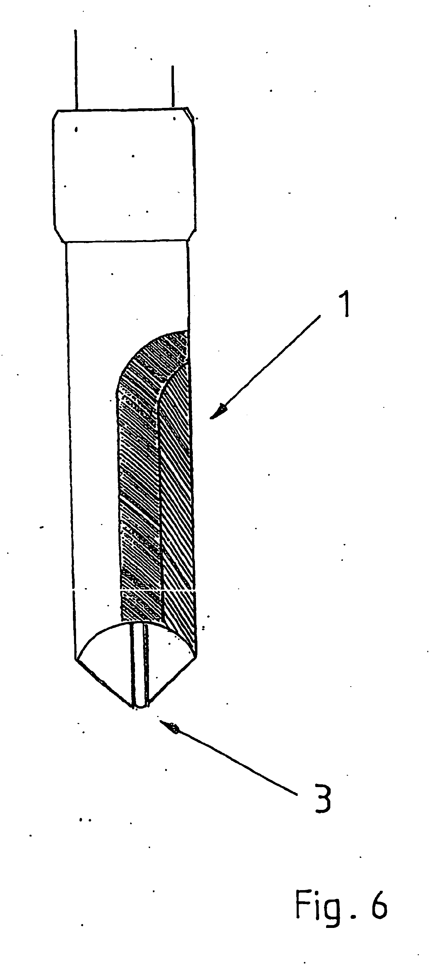

In Fig. 5 ist der Implantatbettvorbohrer 1 mit ausgefahrener Fühlerspitze 3 gezeigt und Fig. 6 zeigt den Implantatbettvorbohrer der Fig. 5 in vergrößerter Darstellung.

In den Fig. 5 und 6 ist der "Schnellstoppzustand" des Bohrers gezeigt Bricht nämlich plötzlich während des Bohrvorganges die Widerstandskraft des vor der Fühlerspitze 3 liegenden Gewebes zusammen, z.B. beim Durchdringen der kieferhöhlenseitigen Corticalis, so wird die Fühlerspitze 3 aufgrund des größeren Innendruckes aus dem Bohrer 1 herausgefahren und entkoppelt hierdurch einerseits den Bohrer 1 mechanisch vom Antrieb und andererseits wird sofort der Strom ausgeschaltet und der Bohrer wird aktiv zusätzlich gebremst, so dass er hierdurch so rechtzeitig gestoppt wird, dass er nicht die Schneider'schen Membran durchdringen kann. Hierdurch ist das erfindungsgemäße Bohrer-System unabhängig von der Reaktionsschnelligkeit des Operateurs und es wird vermieden, dass obwohl der Operateur das Instrument beim Bemerken des geringeren Widerstandes zurückzieht, dieses dennoch durch den Bohrerauslauf tief in die Kieferhöhle eintritt und hierbei die Schneider'sche Membran penetriert.

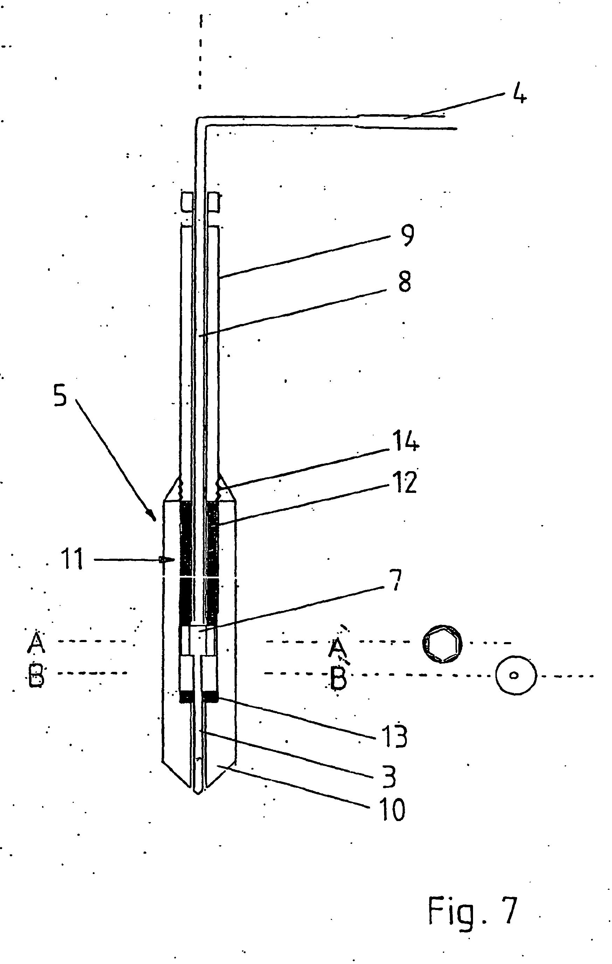

Fig. 7 zeigt einen detaillierten Längsschnitt durch einen Bohrer 1 im belasteten Zustand, d.h. mit eingefahrener Fühlerspitze 3. Die Fühlerspitze 3 ist im belasteten und damit eingefahrenen Zustand mit dem Vorbohrergehäuse 5 mechanisch gekoppelt, so dass sie beim Bohren mit der Vorbohrerspitze 10 mitdreht. Im Beispielsfalle erfolgt die mechanische Kopplung dadurch, dass das distale Ende 6 der Fühlerspitze 3 als Außensechskant ausgebildet ist, welches in ein als Innensechskant ausgebildetes Antriebsteil 7 des Implantatbettvorbohrers 1 im belasteten Zustand eingreift. Über das Druckaufbau- und Druckmesssystem 4 wird mittels physiologischer NaCl-Lösung ein definierter Schwellenwertdruck aufgebaut, welcher über eine an ihrem distalen Ende gewinkelte Kanüle 8, die durch einen Schaft 9 des Implantatbettvorbohrers 1 in dessen Inneres führt und in den Innensechskant-förmigen Antriebsteil 7 mündet. Befindet sich die Fühlerspitze 3 in dichtem Gewebe, welches eine Widerstandskraft aufweist, die größer oder gleich der Schwellenwertkraft (Innendruck) ist, hält der Außensechskant des distalen Endes 6 der längsverschieblichen Fühlerspitze 3 dem am Antriebssechskant 7 liegenden Innendruck das Gleichgewicht, so dass Fühlerspitze 3 und Vorbohrerspitze 10 miteinander um die Kanüle 8 rotieren. Dabei ist die innengeführte Kanüle 8 gegen die Außenwand 11 des Vorbohrergehäuses 5 mittels einer Dichtung 12 abgedichtet. Die Abdichtung in proximaler Richtung erfolgt mittels einer Dichtung 13, welche ringförmig um die Fühlerspitze 3 angeordnet ist.

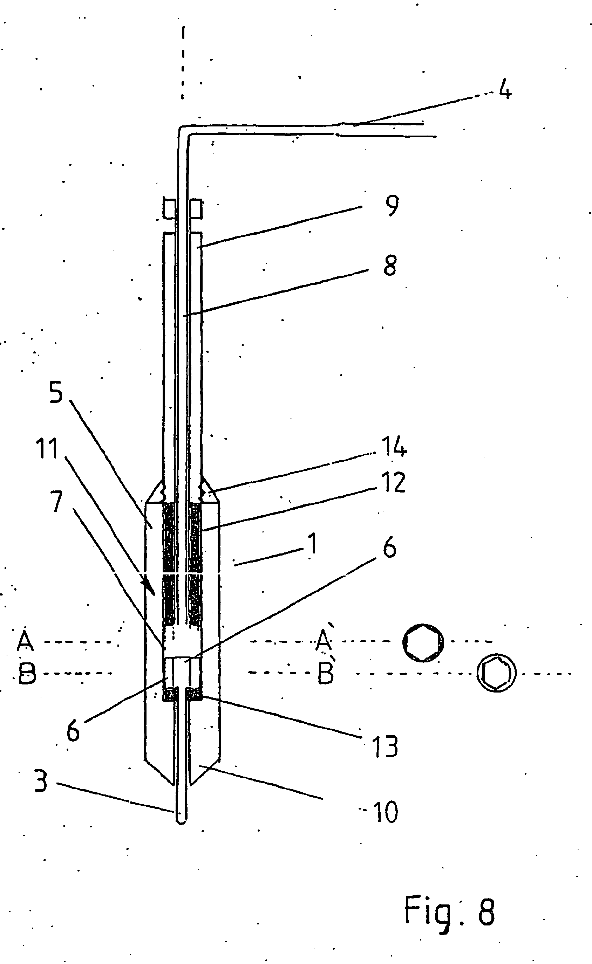

Der Schaft 9 des Implantatbettvorbohrers 1 ist mit dem Vorbohrergehäuse 5 über ein Gewinde 14 verschraubt. Der entsprechende Implantatbettvorbohrer im Ruhezustand ist in Fig. 8 gezeigt: Sobald die Fühlerspitze 3 in weniger dichtes Medium vordringt, entlädt sich der über das zuführende sensorische Druckaufbausystem 4 aufgebaute Druck und die Fühlerspitze 3 bewegt sich schneller nach vorne (vgl. Fig. 8) als das Vorbohrergehäuse 5. Damit wird die Fühlerspitze 3 von der Drehbewegung entkoppelt, weil Innen- und Außensechskant voneinander getrennt werden, der Druck im sensorischen Druckaufbausystem 4 fällt ab und löst über ein in den Fig. 7 und 8 nicht gezeigtes Regelsystem den Stillstand der Drehbewegung auch des Vorbohrergehäuses 5 und damit den Stillstand der Bohrerspitze 10 aus. Zur weiteren Verdeutlichung der mechanischen Kopplung und Entkopplung von Antriebsinnensechskant 6 und Außensechskant 7 der Fühlerspitze 3 sind in den Fig. 7 und 8 die Querschnitte beider Teile 6 und 7 entlang den Schnittlinien A---A' und B---B' gezeigt.

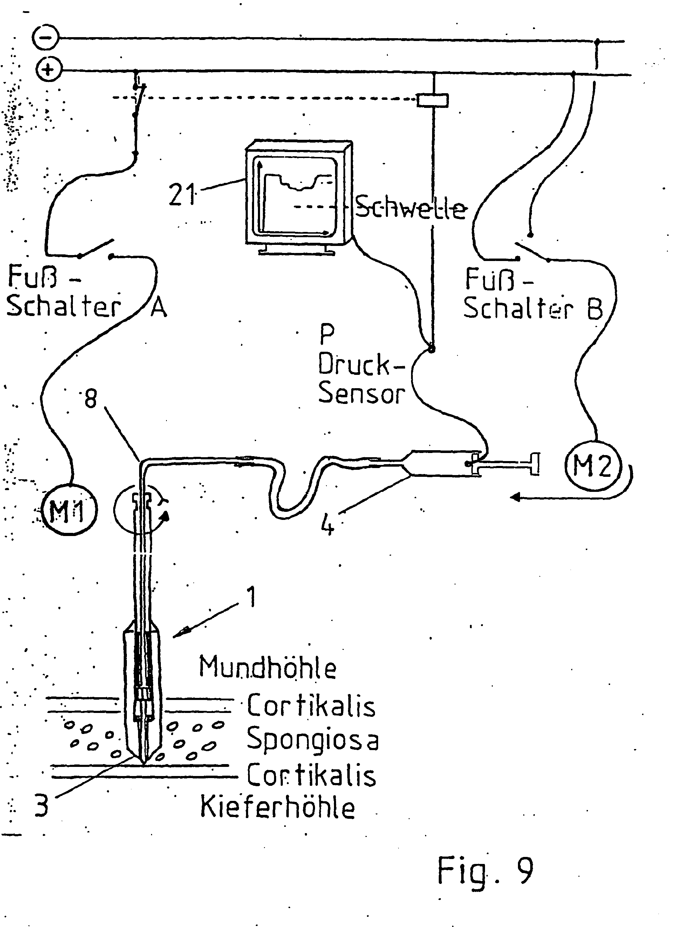

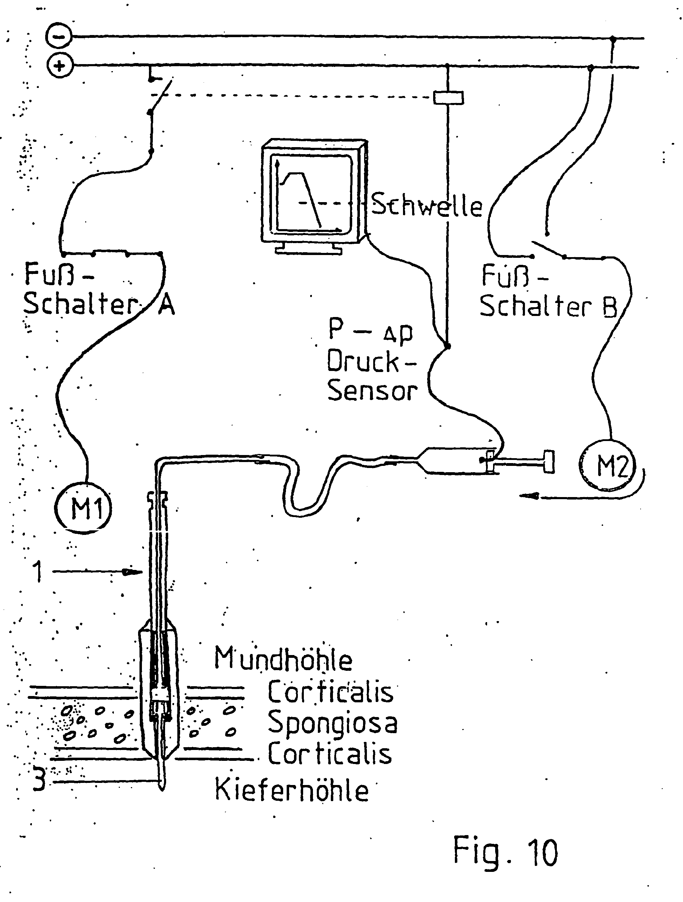

Anhand von Röntgenbildern kann der Operateur die Dichte des Knochens klassifizieren und das sensorische Druckaufbausystem 4 über die Voreinstellung des Anfangsdruckes im sensorischen System kalibrieren und anpassen. In den Figuren 9 und 10 ist in schematischer Darstellung das Gesamtsystem gezeigt, wobei Fig. 9 den Implantatbettvorbohrer 1 und dessen eingefahrene Fühlerspitze 3 im Arbeitszustand zeigt, wobei das proximale Ende der Fühlerspitze 3 kurz vor der kieferhöhlenseitigen Corticalis liegt. Fig. 10 zeigt den Implantatbettvorbohrer 1 im ausgelösten Stillstand mit ausgefahrener Fühlerspitze 3, welche sich bereits innerhalb der Kieferhöhle befindet.

Es ist bevorzugt, dass der Bohrerantriebsmotor M1 mittels eines Fussschalters A und der Pumpenmotor M2, welcher im Beispielsfalle als Rohrmotor ausgebildet ist, mittels eines Fussschalters B betätigt werden kann. Die relevanten Schwellenwertdruck- und IST-Wertdaten werden auf einem Monitor 21 graphisch dargestellt.

Über einen Drucksensor P wird der Druck innerhalb des Druckaufbau- und Druckmesssystems 4 erfasst. Fällt er unter einen vorbestimmten Schwellenwert, so schaltet der Regler R sofort die Stromzufuhr für den Antriebsmotor M1 des Implantatbettvorbohrers 1 ab. Zusätzlich wird der Antriebsmotor M1 im Beispielsfalle induktiv gebremst, so dass der Implantatbettvorbohrer 1 quasi sofort zum Stillstand kommt.

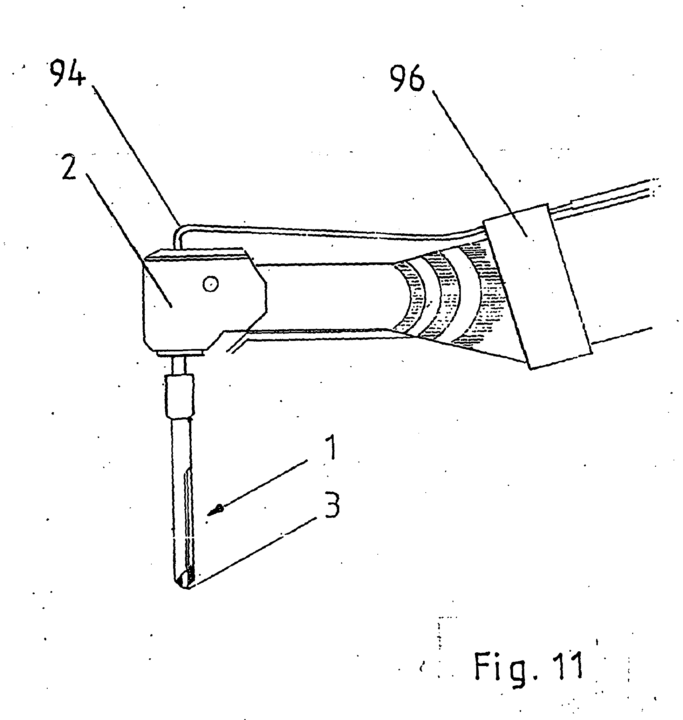

In den Figuren 11 bis 14 ist eine Ausführungsform der vorliegenden Erfindung dargestellt. Wie in der voranstehend bereits beschrieben, ist ein Implantatbettvorbohrer 1 vorgesehen, in welchem axial verschiebbar eine Fühlerspitze 3 angeordnet ist. Die Fühlerspitze 3 kann ihrerseits wiederum einen axial verlaufenden Kanal zur Zufuhr von Kühl- und/oder Spülflüssigkeit aufweisen. Selbstverständlich kann diese Flüssigkeit auch von extern zugeführt werden.

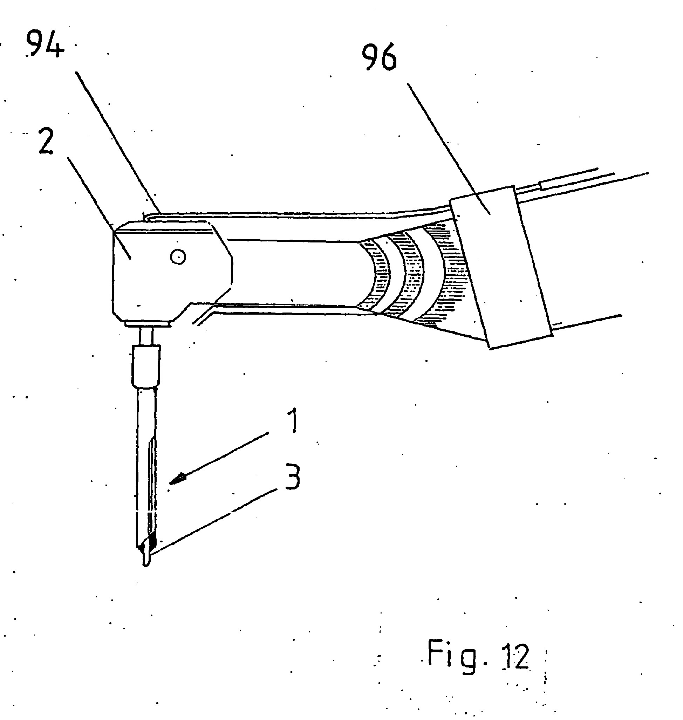

Die Fühlerspitze 3 erstreckt sich proximal durch den Winkelstück-Triebkopf 2 zu dessen Außenseite hin und geht hier in einen im wesentlichen um 90°C zur axialen Verlaufsrichtung der Fühlerspitze 3 verlaufenden Abschnitt 94 über. Die Fühlerspitze 3 und der Abschnitt 94 können einstückig ausgebildet sein. Der endseitige Teil des Abschnitts 94 ist im Bereich des Winkelstück-Triebkopfes festgelegt, beispielsweise durch eine Gummimanschette 96. Man erkennt aus der zeichnerischen Darstellung insbesondere der Figuren 11 und 12, dass durch die gewählte Anordnung sich die Fühlerspitze 3 gegenüber dem Implantatbettvorbohrer 1 axial bewegen kann, wobei der quasi als Ausleger wirkende Abschnitt 94 auf- und abbewegbar ist, wobei weiterhin sein proximales Ende durch die Manschette 96 gegenüber dem Triebkopf 2 festgelegt ist, so dass der Abschnitt 94 auf die Fühlerspitze eine nach distal gerichtete elastische Vorspannung aufbringt.

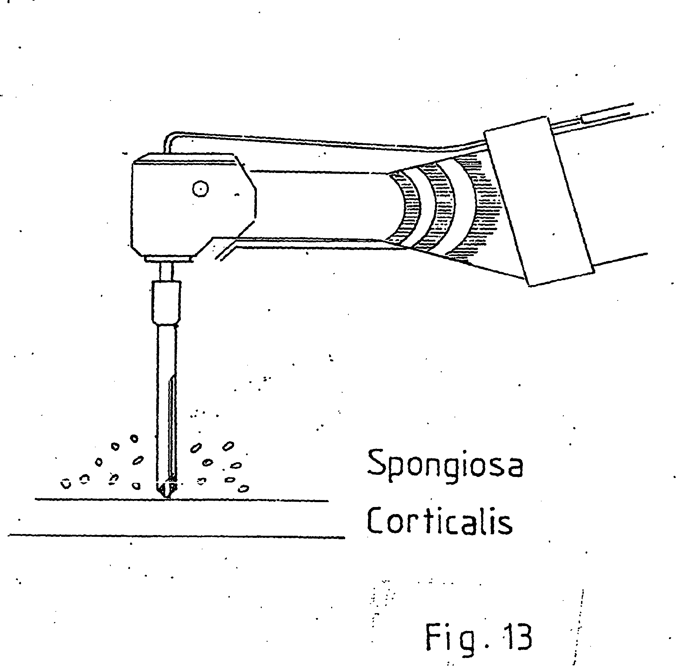

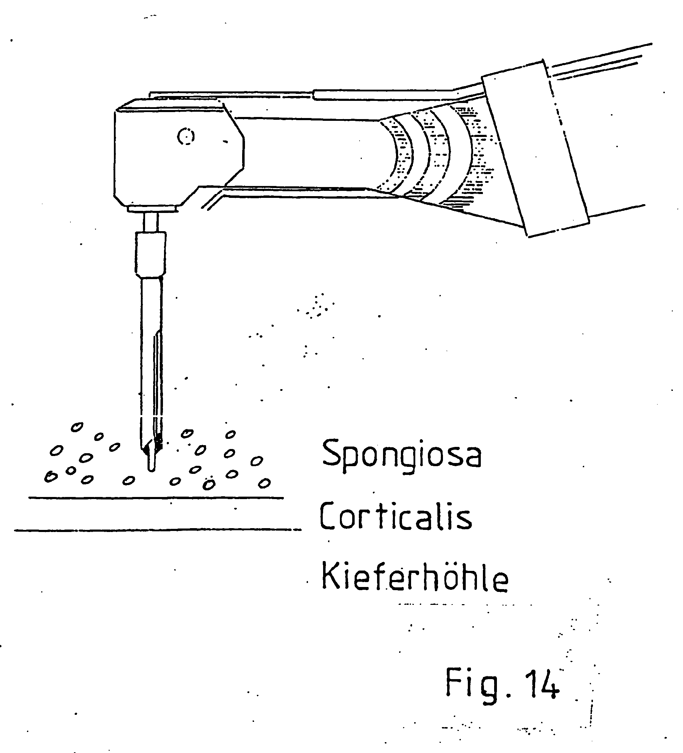

Die erfindungsgemäße Ausführungsform verwendet gerade diese Auslenkung oder Relativbewegung des Abschnittes 94 gegenüber dem Winkelstück-Triebkopf 2, um dem Operateur anzuzeigen, dass im Zuge des Bohrvorganges ein Wechsel von hartem zu weichem Gewebe oder Knochenmaterial bzw. umgekehrt vorliegt, so dass der Bohrvorgang rechtzeitig abgebrochen werden kann: trifft die Fühlerspitze 3 auf festen Widerstand, wird der elastisch verformbare Abschnitt 94 vom Triebkopf 2 aus nach oben ausgelenkt, wie in den Figuren 11 und 13 gezeigt. Es können an dem koaxial zum Bohrer 1 verlaufenden Abschnitt der Fühlerspitze 3 im Bereich der rechtwinkligen Abbiegung Markierungen angebracht werden, beispielsweise sich farblich unterscheidende Ringe, ringförmig umlaufende Einkerbungen etc., welche sukzessive aus der oberen Endfläche des Triebkopfes 2 austreten und damit sichtbar werden und welche es dem Operateur ermöglichen, den Widerstandsgrad des soeben zu bearbeitenden Gewebe- oder Knochenmaterials zu beurteilen. Je höher der Widerstand ist, umso weiter wird der Abschnitt 94 aus dem Triebkopf 2 heraus nach oben verdrängt bzw. ausgelenkt. Trifft die Fühlerspitze 3 auf nachgiebiges oder weiches Gewebe oder Knochenmaterial, federt der Abschnitt 94 aufgrund seiner inhärenten Federkraft wieder in Richtung des Triebkopfes 2 zurück, wie in den Figuren 12 und 14 gezeigt: dies ist ein eindeutiges Indiz für den Operateur, dass der Bohrer 1 unmittelbar davor ist, weiches Gewebe oder Knochenmaterial zu erreichen bzw. zu penetrieren.

Auf rein mechanische und kostengünstige Weise kann somit durch die Ausgestaltungsform der vorliegenden Erfindung dem Operateur zuverlässig übermittelt werden, wann die Spitze des Bohrers 1 unmittelbar davor ist, in die Kieferhöhle einzudringen.

An dem Abschnitt 94 zwischen dem Triebkopf 2 und der Manschette 96 können im Bedarfsfall noch zusätzliche sensorische Elemente angebracht werden, beispielsweise Mikroschalter, welche bei einem Zurückfedern des Abschnittes 94 gemäß den Figuren 12 und 14 den Antriebsmotor für den Bohrer 1 abschalten, eine beispielsweise induktiv oder auf dem Wirbelstromprinzip basierende Abbremsung des Bohrers 1 vornehmen, als Endlagensensoren dienen, welche beispielsweise ein zu rasches Vordringen des Bohrers 1 angeben etc. Weiterhin können in besagtem Abschnitt 94 Dehnungsmessstreifen angeordnet werden, welche eine auf einem Monitor anzeigbare Aussage über das momentane Bohrverhalten oder den Bohrfortschritt liefern.

Es sei an dieser Stelle ausdrücklich darauf hingewiesen, dass die unter Bezug auf die Figuren 3 bis 10 erwähnten Ausgestaltungen, Modifikationen und Zusatzeinrichtungen auch bei der vorliegenden Erfindung anwendbar sind, beispielsweise was die Zufuhr von Kühl- oder Spülflüssigkeit entweder über das Innere des Bohrers 1 oder der Fühlerspitze 3 oder auch von außen her, die Druckbeaufschlagung (Schwellenwerteinstellung)mittels eines Fluids etc. betrifft. Zur Vermeidung von Wiederholungen sei dies an dieser Stelle explizit angeführt. Mit anderen Worten, die unter Bezug auf die obige, an sich nicht zum Gegenstand der vorliegenden Erfindung gehörende Ausführungsform gemachten Ausführungen treffen - soweit technisch sinnvoll - in adäquater Weise auf die erfindungsgemäße Ausführungsform gemäß den Figuren 11 bis 14 zu.

## Patentansprüche

1. Bohrkopf (2) mit Antriebs- und Kühlmitteln und mit einem Bohrer, insbesondere zum Knochenfräsen zur Vorbereitung des Einsetzens von Zahnimplantaten, der in den Bohrkopf (2) eingesetzt ist, wobei
- der Bohrer (1) eine äußere Bohrerspitze (10) aufweist, in deren Innerem eine koaxial gelagerte Fühlerspitze (3) angeordnet ist;
- die Fühlerspitze (3) in axialer Richtung aus der Spitze des Bohrers (1) ausschiebbar ist; und
- die Fühlerspitze (3) mit einer vorbestimmten einstellbaren Schwellenwertkraft in Ausschiebrichtung beaufschlagt ist, so dass die Fühlerspitze (3) bei Unterschreiten einer der Schwellenwertkraft entgegen gerichteten Widerstandskraft aus der äußeren Bohrerspitze (10) heraustritt,
**dadurch gekennzeichnet, dass**
ein der Bohrerseite abgewandtes Ende (94) der Fühlerspitze (3) aus dem Bohrkopf (2) herausgeführt und zusammen mit dieser gegenüber dem Bohrkopf (2) in visuell erkennbarer Weise bewegbar ist und Hierdurch einem Operateur den Übergang von hartem zu weichem Knochen- oder Gewebematerial und umgekehrt anzeigt.

2. Bohrkopf nach Anspruch 1, **dadurch gekennzeichnet, dass** die Schwellenwertkraft der Fühlerspitze (3) durch eine Druckbeaufschlagung mittels eines Fluids erfolgt.

3. Bohrkopf nach Anspruch 2, **dadurch gekennzeichnet, dass** das Fluid eine flüssigkeit, insbesondere eine physiologische Kochsalzlösung ist.

4. Bohrkopf nach Anspruch 2 oder 3, **dadurch gekennzeichnet, dass** die Druckbeaufschlagung mittels einer Kolbenpumpe erfolgt.

5. Bohrkopf nach "Anspruch 1, **dadurch gekennzeichnet, dass** die Schwellenwertkraft der Fühlerspitze (3) durch eine dem abgewandte Ende (94) inhärente elastische Rückstellkraft erzeugt wird.

6. Bohrkopf nach einem der Ansprüche 1 bis 5, **dadurch gekennzeichnet, dass** das der Bohrerseite abgewandte Ende (94) der Fühlerspitze (3) beim Hineindrängen der Fühlerspitze (3) in die äußere Bohrerspitze (10) aus dem Bohrkopf (2) heraustritt und umgekehrt beim Herausschieben der Fühlerspitze (3) aus der äußeren Bohrerspitze (10) in den Bohrkopf (2) eintritt.

## Claims

1. Drilling head (2) with device for propulsion and cooling and with a drill, in particular used when bone has to be preparatorily milled before insertion of dental implants, set into the drilling head, hereby
- the drill (1) exhibits at its external end a drilling-tip (10) with a central coaxially beard sensitive tip
- the sensitive tip (3) can be moved axially towards the external end of the drilling-tip
the sensitive tip (3) is driven towards its external end by a force at a predefined and adjustable threshold, so that the sensitive tip (3) exceeds over the external drilling tip (10) when an opposite directed resistance-force does not reach the threshold
**characterized** as
the other end of the sensitive tip (94), situated opposite and more distant to the drilling part, overviews the drilling head (2) and is in accordance to the sensitive tip (3) moveable relative to the drilling head (2), so visualizing this movement and indicating the transition from hard to soft bone- or tissue-material and reverse to the operator

2. Drilling head according claim 1, **characterized** as the threshold-force upon the sensitive tip (3)is generated by a fluid.

3. Drilling head according claim 2, **characterized** as the fluid is a liquid solution, particular a solution of physiological Natriumchloride.

4. Drilling head according claims 2 or 3, **characterized** as the force is generated by pressure from a piston-pump.

5. Drilling head according claim 1, **characterized** as the threshold-force to the sensitive tip (3) is generated by an elastic redeforming force, which is inherent to its distant end (94)

6. Drilling head according one of the claims 1 to 5, **characterized** as the relative to the drilling part opposite and more distant situated end (94) of the sensitive tip (3), exceeds over the drilling head (2) when the sensitive tip (3) is forged into the external drilling tip (10) and reverse moves into the drilling head (2)when the sensitive tip (3) exceeds over the drilling tip (10).

## Revendications

1. Une tête de fraise (2) avec des moyens de transmission et de refroidissement et avec une fraise placée dans la tête de fraise (2), en particulier pour fraiser l'os en préparant la mise en place d'implants dentaires, étant donné
- que la fraise (1) présente une pointe extérieure de la fraise (10) qui est munie d'une sonde (3) posée coaxialement à son intérieur;
- que la sonde (3) peut être sortie de la pointe de la fraise (1) en direction axiale; et
- que la sonde (3) est munie d'une valeur seuil en direction de la sortie réglable d'avance de manière que la sonde (3) sorte de la pointe extérieure de la fraise (10) quand on atteint une valeur au dessous d'une résistance contraire à la valeur seuil,
tout cela **se caractérisant par le fait**
**qu'**un bout (94) de la sonde (3) à l'opposé de la fraise est sorti de la tête de fraise (2) et, avec la sonde, peut être bougé visuellement perceptible ce qui indique au chirurgien la transition entre le matériel d'os ou de tissu dur au matériel mou et vice versa.

2. Une tête de fraise selon le dépôt n°1, **caractérisée par le fait que** la valeur seuil de la sonde (3) se produit par une injection de pression au moyen d'un fluide.

3. Une tête de fraise selon le dépôt n° 2, **caractérisée par le fait que** le fluide est un liquide, en particulier une solution sodique physiologique.

4. Une tête de fraise selon les dépôts n° 2 ou 3, **caractérisée par le fait que** l'injection de pression est effectuée à l'aide d'une pompe à piston.

5. Une tête de fraise selon le dépôt n° 1, **caractérisée par le fait que** la valeur seuil de la sonde (3) est produite par une force de rétablissement élastique, inhérente au bout opposé (94).

6. Une tête de fraise selon les dépôt n° 1 à 5, **caractérisée par le fait que** le bout (94) de la sonde (3) à l'opposé de la fraise sort de la tête de fraise (2) lors de l'entrée de la sonde (3) dans la pointe extérieure de la fraise (10) et, vice versa, entre dans la tête de fraise (2) lors de la sortie de la sonde (3) de la pointe de fraise extérieure(10).
